(19) Europäisches Patentamt

European Patent Office

Office européen des brevets

(11) **EP 1 002 508 A2**

(12) **EUROPÄISCHE PATENTANMELDUNG**

(43) Veröffentlichungstag:
   24.05.2000 Patentblatt 2000/21

(51) Int. Cl.<sup>7</sup>: **A61F 13/14**

(21) Anmeldenummer: **99122433.8**

(22) Anmeldetag: **10.11.1999**

(84) Benannte Vertragsstaaten:
   **AT BE CH CY DE DK ES FI FR GB GR IE IT LI LU MC NL PT SE**
   Benannte Erstreckungsstaaten:
   **AL LT LV MK RO SI**

(30) Priorität: **18.11.1998 DE 19853252**

(71) Anmelder:
   **Beiersdorf Aktiengesellschaft**
   **20245 Hamburg (DE)**

(72) Erfinder:
   • **Himmelsbach, Stefanie**
      **21614 Buxtehude (DE)**
   • **Himmelsbach, Peter**
      **21614 Buxtehude (DE)**

Bemerkungen:
   Ein Antrag gemäss Regel 88 EPÜ auf Hinzufügung des Anspruchs Nr.8 liegt vor. Über diesen Antrag wird im Laufe des Verfahrens von der Prüfungsabteilung eine Entscheidung getroffen werden (Richtlinien für die Prüfung im EPA, A-V, 3.).

(54) **Selbstklebend ausgerüstetes Stillpflaster**

(57)    Selbstklebend ausgerüstetes Stillpflaster zur Aufnahme von fetthaltigen wäßrigen Gemengen, bestehend aus einem saugfähigen Kern, der wenigstens teilweise mit einer feuchtigkeitsabweisenden Trennsicht überzogen ist, wobei das Stillpflaster einseitig partiell mit einer Selbstklebemasse ausgerüstet worden ist, so daß das Stillpflaster eine Klebkraft von mindestens 1 N/cm aufweist.

Figur 1

EP 1 002 508 A2

**Beschreibung**

Die Erfindung betrifft ein selbstklebend ausgerüstetes Stillpflaster, welches zumindest partiell mit einer Selbstklebemasse beschichtet ist und welches aus saugfähigem Material besteht, um Reste von Muttermilch aufnehmen zu können.

Die Verwendung von Stilleinlagen zur Aufnahme von Muttermilch ist eine verbreitete Praxis. Dabei werden diese in den Büstenhalter der Verwenderin eingelegt werden, um aus den Brüsten heraustropfende beziehungsweise laufende Milch aufsaugen zu können.

Sie verhindern ein Aufweichen der Haut im Brustwarzenbereich und verringern somit die Gefahr der Rißbildung bei Belastung. Aus hygienischen Gründen werden Einwegprodukte bevorzugt.

Insbesondere kommen daher Stilleinlagen zum Einsatz, die aus einem sehr saugfähigem Material bestehen.

Eine derartige Stilleinlage offenbart die DE G 83 09 379. Die Stilleinlage besteht aus drei Schichten, und zwar einem Vlies und einer Schicht aus Zellstoff, auf die eine feuchtigkeitsundurchlässige Schicht mit einem hohen Weichmacheranteil aufgebracht ist. Die bevorzugt rund geformte Stilleinlage kann zur Angleichung an die weibliche Brust tiefgezogen sein.

Das Produkt ist nicht klebend ausgerüstet. Nachteil dieser Einlagen ist u.a. das nicht auszuschließende Verrutschen der Einlage beim Tragen. Beim Verrutschen kann es zum Durchdringen/Verschmutzen der Kleidung und zum Aufweichen der Haut kommen.

Auch die DE 32 06 371 A1 offenbart eine kreisrunde Stilleinlage für den Büstenhalter stillender Mütter, die aus mehreren Schichten aufgebaut ist. Die körperseitige Deckschicht wird von einer feuchtigkeitsdurchlässigen Schicht, vorzugsweise ein Polypropylenvlies, gebildet. Darauf befindet sich eine feuchtigkeitsaufsaugende Schicht aus Watte, die außen von einer wiederum feuchtigkeitsabweisenden Schicht (zum Beispiel ein Vlies oder eine Folie oder spezielle Trennausrüstungen wie Imprägnierungen, Hydrophobierungen oder Oleophobierungen) überzogen ist. Die innere und die äußere (Vlies-)Schicht sind im Randbereich miteinander flachverprägt. Auch bei der Verwendung von Trennfolien lassen sich, obwohl diese unperforiert kaum eine Luftdurchlässigkeit aufweisen, hohe Wasserdampfdurchlässigkeiten von bis zu 3200 g/(24h x m$^2$) erzielen. Bevorzugt werden Durchlässigkeit von mindestens 500 g/(24h x m$^2$), besonders bevorzugt größer 1000 g/(24h x m$^2$) und ferner zwischen 1200 g/(24h x m$^2$) und 3200 g/(24h x m$^2$).

Auf der Außenseite der Stilleinlage kann eine selbstklebende Beschichtung aufgebracht sein, die eine Fixierung der Stilleinlage in einem Büstenhalter sicherstellen soll.

Mit der DE G 82 07 723 ist eine weitere Einlage für einen Stillbüstenhalter bekannt geworden, die aus saugfähigem Material besteht und die innen oder außen mit einen haftklebenden partiellen Beschichtung versehen ist. Die klebenden Abschnitte der Stilleinlage dienen aber lediglich dazu, die Stilleinlage in ihrer Position in dem zwingend erforderlichen Büstenhalter zu fixieren.

Die DE-OS 28 18 162 beschreibt eine trägerlose selbstklebend ausgerüstete Brusthebe, die von einem Formstück aus Kunststoff gebildet wird. Das Formstück, das etwa von der Mitte des Busens bis zum unteren Brustansatz reicht, soll in seinem mittleren Bereich kurvenförmig nach außen geführt werden und zu den seitlichen Enden hin sich ansatzartig verjüngen.

Die Verwendung des vollflächig selbstklebend beschichteten Formstücks ist auf die Stützung der Brust beschränkt.

Nachteilig bei den bekannten Stilleinlagen ist, daß diese an die Verwendung in einem Büstenhalter geknüpft sind. Ein Ablegen des Büstenhalters der stillenden Mutter ist daher weitgehend ausgeschlossen, will sie nicht das Risiko auf sich nehmen, daß sich die Kleidung mit aus der Brust austretenden Milch vollsaugt.

Ein permanentes, ganztägiges Tragen des Büstenhalters ist ebenso unvorteilhaft, da es sehr beengend ist und zu Verspannungen führen kann. Insbesondere in langen Perioden zwischen der Milchabgabe kommt es zu sehr hohen ungeregelten Milchausstößen. Dieses ist insbesondere in der Nacht. Hier wird das Verlangen nach mehr Sicherheit und Sauberkeit angesprochen.

Aufgabe der Erfindung ist es, ein Stillpflaster zur Verfügung zu stellen, welche ohne Büstenhalter getragen werden kann und welche die aus dem Stand der Technik bekannten Nachteile nicht aufweist.

Gelöst wird die Aufgabe durch ein Stillpflaster, wie es im Anspruch 1 dargelegt ist. Die Untersprüche umfassen vorteilhafte Varianten des Erfindungsgegenstands.

Demgemäß betrifft die Erfindung ein selbstklebend ausgerüstetes Stillpflaster zur Aufnahme von fetthaltigen wäßrigen Gemengen, wie sie u.a. von der Muttermilch dargestellt werden. Das Stillpflaster besteht aus einem saugfähigen Kern, zum Beispiel Watte, der wenigstens teilweise mit einer feuchtigkeitsabweisenden Trennsicht überzogen ist, wobei das Stillpflaster einseitig partiell mit einer Selbstklebemasse ausgerüstet worden ist, so daß das Stillpflaster eine Klebkraft von mindestens 1 N/cm aufweist.

Mittig sollte ein erfindungsgemäßes Stillpflaster mindestens partiell eine Luftdurchlässigkeit von mindestens 1 cm$^3$/(cm$^2$ x s) aufweisen, bevorzugt größer 15 cm$^3$/(cm$^2$ x s), besonders bevorzugt zwischen 20 cm$^3$/(cm$^2$ x s) und 120 cm$^3$/(cm$^2$ x s). Weiterhin hat es sich als vorteilhaft erwiesen, wenn das Stillpflaster mindestens partiell eine Wasserdampfdurchlässigkeit von mindestens 500 g/(24h x m$^2$) aufweist, bevorzugt größer 1000 g/(24h x m$^2$), besonders bevorzugt zwischen 1200 g/(24h x m$^2$) und 3200 g/(24h x m$^2$).

Das Stillpflaster nimmt vorzugsweise mindestens 5 g des fetthaltigen wäßrigen Gemenges pro Gramm Eigengewicht auf. Bevorzugt wird die Aufnahme von 7 g bis 30 g des fetthaltigen wäßrigen Gemenges pro Gramm Eigengewicht. In besonders vorteilhaften ausgestatteten Stillpflastern liegt die Aufnahmemenge im Bereich von 8 g bis 25 g des fetthaltigen wäßrigen Gemenges pro Gramm Eigengewicht.

In einer weiteren bevorzugten Ausführungsform weist das Stillpflaster einen Durchmesser von 6 bis 14 cm und eine Dicke von weniger als 15 mm auf.

Weiter vorzugsweise weist das Stillpflaster eine Dehnbarkeit von mindestens 10 % in mindestens eine Richtung auf, und zwar bei einer Belastung von mindestens 3 N/cm. Bevorzugt werden der Bereich 15 % bis 500 % beziehungsweise besonders der Bereich 30 % bis 300 %, weil sich das Stillpflaster dann besser anmodellieren läßt.

Ein weiterer Vorteil kann durch die Kombination mit speziellen Klebemassen erzielt werden. Das selbstklebend ausgerüstete Produkt kann dann durch Zug am selben in der Verklebungsebene entkleben, wodurch ein schmerzfreies Ablösen möglich ist. In einer speziellen Ausführung sind herzu Anfasser am Stillpflaster vorhanden.

Das Stillpflaster kann eine unterstützende stabilisierende Brusthebefunktion ausüben.

Bevorzugt wird, die Selbstklebemasse im Randbereich des Stillpflasters ringförmig aufzubringen, wobei der Ring eine Breite von mindestens 1 mm aufweist.

Bevorzugte Ausführungen haben einen Kleberrand von 1,5 bis 25 mm. Dieser kann darüber hinaus durch Stege unterbrochen sein.

Für die Beschichtung wird eine Selbstklebemasse bevorzugt, welche eine hohe Klebkraft aufweist. Die erfindungsgemäße Stillauflage weist insbesondere eine Klebkraft zwischen 1,5 N/cm und 15 N/cm auf, und zwar bezogen auf die tatsächlich beschichtete Kleberandbreite.

Als Klebemassen lassen sich vorteilhafterweise thermoplastische Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Gegebenenfalls kann eine Nachvernetzung durch UV- oder Elektronenstrahlen-Bestrahlung angebracht sein.

Insbesondere Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Ihr Erweichungspunkt sollte höher als 50 °C liegen, da die Applikationstemperatur in der Regel mindestens 90 °C beträgt, bevorzugt zwischen 120 °C und 150 °C beziehungsweise 180 °C und 220 °C bei Silikonen.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachern und Weichmachern.

Die Klebemasse beinhaltet vorzugsweise mindestens eine aromatische Komponente, welche einen Anteil von weniger als 35 %, bevorzugt 5 bis 30 %, aufweist.

Für besonders starkklebende Systeme basiert die Heißschmelzklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzklebemasse die nachfolgend angegebene Zusammensetzung auf:

| | |
|---|---|
| 10 Gew.-% bis 90 Gew.-% | Blockcopolymere, |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Die Heißschmelzselbstklebemasse weist in einer bevorzugten Ausführungsform einen Erweichungspunkt auf oberhalb von 70 °C, bevorzugt 85 °C bis 140 °C.

Die Heißschmelzselbstklebemassen sind vorzugsweise so eingestellt, daß sie bei einer Frequenz von 0,1 rad/s eine dynamisch-komplexe Glasübergangstemperatur von weniger als 25 °C, bevorzugt von 5 °C bis -30 °C, ganz besonders bevorzugt von 0 °C bis -25 °C, aufweisen.

Insbesondere an medizinische Trägermaterialien werden hohe Anforderungen bezüglich der Klebeeigenschaften gestellt. Für eine ideale Anwendung sollte die Heißschmelzklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzklebemasse notwendig.

Durch die gezielte Absenkung der Glasübergangstemperatur der Klebemasse infolge der Auswahl der Klebrigmacher, der Weichmacher sowie der Polymermolekülgröße und der Molekularverteilung der Einsatzkomponenten wird die notwendige funktionsgerechte Verklebung der Haut mit dem Stillpflaster erreicht.

Die hohe Scherfestigkeit der hier eingesetzten Klebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet. Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzselbstklebemasse zwischen zwei planparallelen Planen mit variablen Frequenzen und geringer Verformung (linear viskoelastischer Bereich) in Schwingungen versetzt. Über eine Aufnahmesteuerung wird computerunterstützt der Quotient ( $Q = \tan \delta$ ) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz.

Besonders für medizinische Anwendungen ist ein relativ niedriger Glasübergangspunkt notwendig.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmelzklebemasse A | -12 ± 2 °C | tan δ = 0,08 ± 0,03 | tan δ = 1,00 ± 0,03 |
| Hesßschmelzklebemasse B | -9 ± 2 °C | tan δ = 0,22 ± 0,03 | tan δ = 1,70 ± 0,03 |

Bevorzugt werden erfindungsgemäß Heißschmelzklebemassen, bei denen das Verhältnis des viskosen Anteils zum elastischen Anteil bei einer Frequenz von 100 rad/s bei 25 °C größer 0,7 ist, oder Heißschmelzselbstklebemassen, bei denen das Verhältnis des viskosen Anteils zum elastischen Anteil bei einer Frequenz von 0,1 rad/s bei 25 °C kleiner 0,4 ist, bevorzugt zwischen 0,35 und 0,02, ganz besonders bevorzugt zwischen 0,3 und 0,1.

Vorteilhaft ist weiterhin, wenn die Klebemasse partiell auf dem Trägermaterial aufgetragen ist, beispielsweise durch Rasterdruck, Thermosiebdruck, Thermoflexodruck oder Tiefdruck, denn im Vollstrich klebend ausgerüstete Stillpflaster können unter ungünstigen Voraussetzungen bei der Applikation mechanische Hautirritationen hervorrufen.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Die Klebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des Themosiebdrucks besteht in der Verwendung einer rotierenden beheizten, nahtlosen, trommelförmigen perforierten Rundschablone, die über eine Düse mit der bevorzugten Heißschmelzselbstklebemasse beschickt wird. Eine speziell geformte Düsenlippe (Rund- oder Vierkantrakel) preßt die über einen Kanal eingespeiste Heißschmelzklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die Heißschmelzklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Klebemasse und die innere Kohäsion des Hotmelts wird von der auf dem Träger bereits haftenden Basis der Kalotten der in den Löchern begrenzte Vorrat an Heißschmelzklebemasse konturenscharf abgezogen beziehungsweise durch den Rakeldruck auf den Träger gefördert.

Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Auflagen, der mit größerem Durchmesser und größerer Kalottenhöhe für rauhe oder stark porige Auflagen vorgesehen ist.

Die Positionierung der Kalotten auf dem Stillpflaster wird durch die in weiten Grenzen variierbare Geometrie des Auftragswerkes, zum Beispiel Gravur- oder Siebgeometrie, definiert festgelegt. Eine Nachbehandlung, insbesondere ein Kalander oder ein Verformen der Klebemassenkalotten, kann zur gezielten Einstellung von Klebeeigenschaften führen.

Das Pflaster wird bevorzugt mit einer Geschwindigkeit von größer 2 m/min, bevorzugt 20 bis 200 m/min, beschichtet, wobei die Beschichtungstemperatur größer als die Erweichungstemperatur zu wählen ist.

Die Heißschmelzklebemasse kann mit einem Flächengewicht von größer 5 $g/m^2$, bevorzugt zwischen 10 $g/m^2$ und 400 $g/m^2$, ganz besonders bevorzugt zwischen 30 $g/m^2$ und 300 $g/m^2$, auf dem Stillpflaster aufgetragen sein, und zwar jeweils bezogen auf die tatsächlich beschichtete Fläche.

Die Kombination der Heißschmelzklebemasse und der bevorzugten ringförmigen Beschichtung gewährleistet auf der einen Seite eine sichere Verklebung, auf der anderen Seite sind zumindest visuell erkennbare allergische oder mechanische Hautimitationen ausgeschlossen.

Der Massetransfer auf die Haut ist aufgrund der hohen Kohäsivität des Klebers vernachlässigbar, weil der Kleber nicht an der Haut verankert, vielmehr ist die Verankerung der Klebemasse auf dem Stillpflaster mit bis zu 25 N/cm (Probenbreite) für medizinische Anwendungen gut.

Je nach Trägermaterial und dessen Temperaturempfindlichkeit kann die Heißschmelzklebemasse direkt aufgetragen sein oder zuerst auf einen Hilfsträger aufgebracht und dann auf die endgültige Auflage transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Weiterhin kann eine Behandlung der Heißschmelzklebemasse mit einer Elektronenstrahl-Nachvernetzung oder einer UV-Bestrahlung zu einer Verbesserung der gewünschten Eigenschaften führen.

Schließlich kann das Stillpflaster nach dem Beschichtungsvorgang mit einem klebstoffabweisenden Trägermaterial, wie silikonisiertes Papier, eingedeckt werden.

Des weiteren kann das Stillpflaster einen oder mehrere Wirkstoffe beinhalten. Unter Wirkstoffen sind alle pharmazeutischen oder kosmetischen Substanzen und Stoffe des Stand der Technik zu verstehen, welche durch Übertragung oder Aufnahme nach der Applikation des Pflasters eine Aktivität übernehmen. Insbesondere hautpflegende und schmerzlindernde Substanzen sind bevorzugt zu verwenden.

Im folgenden soll das erfindungsgemäße Stillpflaster in einer besonders vorteilhaften Ausführungsform anhand einer Zeichnung näher erläutert werden, ohne damit die Erfindung unnötig einschränken zu wollen.

Es zeigt

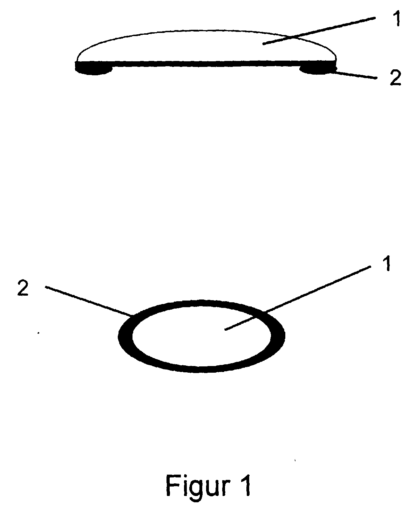

Figur 1   das erfindungsgemäße Stillpflaster in der seitlichen Darstellung sowie in der Aufsicht von unten.

Demgemäß besteht das erfindungsgemäße Stillpflaster aus einem saugfähigen Kern 1, der von Watte gebildet wird. Der Kern 1 ist am Rand ringförmig mit einer selbstklebenden Beschichtung 2 versehen, die die Haftung des Stillpflasters auf dem die weibliche Brust umgebenden Hautbereich sicherstellt.

Um das Stillpflaster von der Brust zu entfernen, kann an diesem in Richtung der Verklebungsebene gezogen werden, so daß es zu einer Entklebung kommt. Um diesen Vorgang zu erleichtern, kann am Kern 1 ein kleiner Anfasser angebracht sein.

**Patentansprüche**

1. Selbstklebend ausgerüstetes Stillpflaster zur Aufnahme von fetthaltigen wäßrigen Gemengen, bestehend aus einem saugfähigen Kern, der wenigstens teilweise mit einer feuchtigkeitsabweisenden Trennsicht überzogen ist, wobei das Stillpflaster einseitig partiell mit einer Selbstklebemasse ausgerüstet worden ist, so daß das Stillpflaster eine Klebkraft von mindestens 1 N/cm aufweist.

2. Stillpflaster nach Anspruch 1, dadurch gekennzeichnet, daß das Stillpflaster mindestens 5 g des fetthaltigen wäßrigen Gemenges pro g Eigengewicht aufnimmt.

3. Stillpflaster nach den Ansprüchen 1 und 2, dadurch gekennzeichnet, daß das Stillpflaster einen Durchmesser von 6 bis 14 cm und eine Dicke von weniger als 15 mm aufweist.

4. Stillpflaster nach den Ansprüchen 1 bis 3, dadurch gekennzeichnet, daß das Stillpflaster eine Dehnbarkeit von mehr als 10 %, bevorzugt 15 bis 500 %, besonders bevorzugt 30 % bis 300 %, aufweist.

5. Stillpflaster nach den Ansprüchen 1 bis 4, dadurch gekennzeichnet, daß das Stillpflaster durch Dehnen entklebbar ist.

6. Stillpflaster nach mindestens einem der vorangegangenen Ansprüche, dadurch gekennzeichnet, daß die Selbstklebemasse im Randbereich des Stillpflasters ringförmig aufgebracht ist, wobei der Ring eine Breite von mindestens 1 mm aufweist, bevorzugt 1,5 mm bis 25 mm.

7. Stillpflaster nach mindestens einem der vorangegangenen Ansprüche, dadurch gekennzeichnet, daß das Stillpflaster einen Wirkstoff beinhaltet.

Figur 1